# EUROPEAN PATENT APPLICATION

(11) **EP 3 560 479 A1**
(43) Date of publication of application: **30.10.2019**
(21) Application number: 19170518.5
(22) Date of filing: 23.04.2019
(51) Int. Cl.: A61K 8/55, A61K 8/63, A61Q 17/04, A61K 8/85, A61K 8/02, A61K 8/9789

(54) **A UV FILTER BASED ON POLYHYDROXYBUTYRATE AND A METHOD OF ITS PREPARATION**

(30) Priority: 24.04.2018 CZ 20180197
(71) Applicant: NAFIGATE Corporation, a.s., 190 00 Praha 9 (CZ)
(72) Inventor: MAROVA, Ivana, 635 00 Brno (CZ); PAVELKOVA, Renata, 669 02 Znojmo (CZ); KUNDRAT, Vojtech, 702 00 Ostrava (CZ); MATOUSKOVA, Petra, 675 03 Budisov (CZ)
(74) Representative: Musil, Dobroslav

(57) **Abstract**

The invention relates to a UV filter based on polyhydroxybutyrate containing polyhydroxybutyrate particles in size ranging from 0.2 to 500 µm, which are functionalised by at least one organic UV filter of natural origin at a total concentration of 2 to 10%.

The invention also relates to the method of preparation of this UV filter.

## Description

### Technical field

The invention relates to a UV filter based on polyhydroxybutyrate.

The invention further relates to a method of preparation of this UV filter based on polyhydroxybutyrate.

### Background art

UV radiation is an electromagnetic radiation with a wavelength ranging from 100 to 400 nm. The UV radiation is divided into three types of UV radiation depending on its wavelength and its biological effects:
UVA radiation with a wavelength ranging from 320 to 400 nm represent about 99% of the UV radiation which reaches the Earth's surface. This radiation penetrates deeper into the skin, induces pigmentation and erythema and affects the deepest structures of the corium (immunosuppressive effect, acceleration of the actin aging process). Besides this, it also generates very reactive oxygen species that indirectly damage the DNA. UVA radiation produced artificially is used, for example, in solariums and in dermatological phototherapy.
UVB radiation with a wavelength ranging from 290 to 320 nm represent about 1% of the UV radiation which reaches the Earth's surface. These rays are carcinogenic and are able to degrade or impair proteins or other vital organic compounds and also to directly damage the DNA through the formation of pyrimidine dimers. Artificially produced UVB radiation is used, for example, in dermatological phototherapy.
UVC radiation, with a wavelength ranging from 100 to 290 nm, do not substantially reach the Earth's surface as it is inhibited by the oxygen of the atmosphere upon production of ozone. This radiation is proven to be lethal for living organisms - it is carcinogenic.
UV radiation is absorbed in the human skin by a variety of chromophores, such as melanin, DNA, RNA, proteins, aromatic amino acids (e.g., tyrosine and tryptophan), lipids, water, and others. The absorption of the UV radiation by these chromophores leads to various photochemical reactions and secondary interactions involving reactive particles (of oxygen and nitrogen) that may have harmful effects. Despite the fact that human skin itself has a number of antioxidants and enzymes that repair the damaged DNA, UV radiation can still cause damage to the skin mutations and genetic instability. For this reason, various protective substances are being developed against UV radiation and its negative effects (including accelerated aging of the skin), which are most often added to various cosmetic products - sun lotions, creams, facial masks, etc., most often in a concentration of 0.1 to 10%.

Nowadays, inorganic (physical) and organic (chemical) UV filters are available. Inorganic UV filters reflect and scatter the light, whereby both these phenomena take place within a wide spectrum of wavelengths from visible light to infrared UV radiation. Inorganic UV filters include, for example, TiO₂ mineral microparticles, which exhibit better protection against UVB radiation or ZnO, which, on the contrary, exhibit a higher protective effect against UVA radiation, etc. These UV filters are photostable, but when used, it is usually necessary to apply a larger layer of the respective cosmetic product to achieve the desired effect. By the miniaturization of the particles of these filters to have a size from 10 to 50 nm the scattering of the visible light is reduced and effective scattering is shifted to lower wavelengths below the UVA spectrum. Such filters are therefore more suitable for being used in cosmetic products. Nevertheless, particles of this size have a higher tendency to aggregate, which can, on the other hand, reduce the efficiency of light scattering. Due to their high photostability, the inorganic UV filters are primarily used to protect children and people allergic to organic UV filters. Organic UV filters, also known as chemical UV filters, absorb the sun radiation and turn it, especially its UVB part, into thermal energy [1]. Their disadvantage is that they absorb the radiation only in a narrow spectrum, and it is usually necessary to combine several of them to provide a product with protection against a wider spectrum of UV radiation. Within the most typical and popular UV organic filters can be mentioned the filters based on cinnamate, including octinoxate and cinoxate or filters based on salicylates, which do not create stains on the skin and only rarely cause irritation. These filters require to be applied more frequently because of their lower efficiency and lower water resistance. Another disadvantage is that they are degraded by the exposure to the sun, which reduces their effectiveness [1].

Beside the use of one type of UV filters, it is also possible to combine organic and inorganic filters within one formulation, whereby these combinations can have a synergistic effect and thus achieve a higher sun protective factor.

The protective factor SPF ("sun protective factor") is used to evaluate the effectiveness of UVB filters. The protective factor SPF is a ratio of the dose of UVB radiation required to create a minimal erythemal (MED - "minimal erythemal dose") on a protected skin versus the dose of UVB radiation required to generate the same effect on an unprotected skin. This factor is determined in vivo. For example, a product with a protective factor 15 filters out 94 % of the UVB radiation, the product with a protective factor 30 then 97 % [2].

A standard EN ISO 24443:2012 test is available to determine the protective factor of cosmetic products against the UVA radiation. In addition to the protective factor, the water resistance of cosmetic products and the water resistance of the UV filters contained are also evaluated [2].

One of the possibilities to extend the effect and the stability of UV filters in cosmetic formulations is to encapsulate these filters into suitable carriers for targeted transport into the human body. Because of the hydrophobic barrier at the surface of the body, the most frequently used carriers are liposomes. They are able to pass through the skin barrier thanks to their hydrophobic surface and can encapsulate various hydrophobic and hydrophilic molecules.

The current trend in cosmetics and in the area of personal protection against UV radiation is the use of natural UV filters, such as carotenoids, some polyphenols, chlorophylls and other natural molecules, often combined with natural antioxidants (such as tocopherol, ascorbate and various mixtures). Natural carriers are also optionally used in order to eliminate as much as possible the utilization of synthetic polymers and the environmental burden associated to this type of molecules. Nowadays, formulations from different types of marine and freshwater algae, for example, are commercially available. Their protective effect against UV radiation is declared together with their positive effect on the skin and their rejuvenating effect. Serious studies aimed at analyzing the real potential of algae for protection against UV radiation and their use in cosmetic products are well summarized, for example, in the reference [2]. In the study [3] is described, for example, the use of a red algae (*Porphyra umbilicalis*) extract in combination with *Ginkgo biloba* extract and vitamins A, C and E, which increases the protective factor of the cosmetic product by 20 %. From the study [4], it is known that algae extract can replace at least part of the synthetic UV filters in a cosmetic product, since it protects DNA against damage and the skin against redness while supporting cell renewal. A suitable source of other active substances useful for protection against UV radiation are, among others, cyanobacteria [5]. For example, cyanobacteria *Scytonema hoffman* growing at high light intensities (300 to more than 2000 µmol.m⁻².s⁻¹), is a source of scytonemin-3a-imine, a newly identified red-mahogany pigment that is considered as a new natural UV filter applicable in cosmetic products [6]. Beside algae and algae extracts, many other natural substances and plant extracts, such as polyphenols and flavonoids, were tested for UV protection. The sources of these active substances include, for example, green tea, Mediterranean milk thistle (*Silybum marianum*), Turmeric (*Curcuma longa*), grapevine, the African tuliptree (*Spathodea campanulata*), caper bush (*Capparis spinosa*), great basil (*Ocimum basilicum*), almond tree (*Prunus amygdalus*), a pea family plant known as Indian beech (*Pongamia pinnata*), and others - see, e.g., [7, 8]. In addition, the effect of the bark of the *Zanthoxylum rhetsa* tree was also actively studied, because it could be used as a natural active ingredient of broad-spectrum tanning formulations and anti-aging formulations [8]. Other complex natural materials with UV filter properties are various types of vegetable oils [9], including green coffee oil [10], roasted coffee oil [11] and even used coffee grounds [12]. The disadvantage of these natural UV filters is that they have to be combined with synthetic UV filters, especially inorganic filters, such as TiO₂ or ZnO, in order to achieve a protective factor 20.

A number of different polymers and composites have been identified and tested as potential carriers of these UV filters, for example, a UV-absorbing nanocomposite based on hydroxyapatite, which absorbs radiation within UV spectrum, and polyvinylpyrrolidone-stabilized ascorbic acid having the advantage of not being cytotoxic [13]. Another variant is, for example, a composite of carboxymethyl-chitosan and pineapple peel carboxymethyl cellulose, which has been tested as a TiO₂ carrier [14], etc.

In addition, a sunscreen gel with hydroxyapatite and chitosan is currently known. It can be supplemented with many active substances, and, at the same time, destroys multi-resistant bacteria [15].

The aim of the invention is to design a UV filter purely based on natural compounds and a method of its preparation.

### References

[1] Trager R.: "US bans microbeads from personal care products" Chemistry Word, January 2016.
[2] Wang H.M.D., Chen Ch.Ch., Hyunh P., Chang J.S.: "Exploring the potential of using algae in cosmetics" Bioresource Technology 184, 2015, pp. 355-362.
[3] Mercurio D.G., Wagemaker T.A.L., Alves V.M. et al: "In vivo photoprotective effects of cosmetic formulations containing UV filters, vitamins, Ginkgo biloba and red algae extracts" Journal of Photochemistry and Photobiology B: Biology 153, 2015, pp. 121-126.
[4] Chiari B.G., Trovati E., Pecoraro E. et al.: "Synergistic effect of green coffee oil and synthetic sunscreen for health care application" Industrial Crops and Products 52, 2014, pp.389-393.
[5] Sinha R.P., Häder D.P.: "UV-protectants in cyanobacteria" Plant Science 174 (3), 2008, pp. 278-289.
[6] Grant C.S., Louda J.W.: "Scytonemin-imine, a mahogany-colored UV/Vis sunscreen of cyanobacteria exposed to intense solar radiation". Organic Geochemistry 65, 2013, pp. 29-36.
[7] Rojas J., Londono C., Ciro Y.: "THE HEALTH BENEFITS OF NATURAL SKIN UVA PHOTOPROTECTIVE COMPOUNDS FOUND IN BOTANICAL SOURCES" International Journal of Pharmacy and Pharmaceutical Sciences 8(3), 2016.
[8] Santhanam R.K., Akhtar M.T., Ahmad S., Abas F., Safinarlsmail I., Rukayadi Y., Shaari K.: Utilization of the ethyl acetate fraction of Zanthoxylum rhetsa bark extract as an active ingredient in natural sunscreen formulations" Industrial Crops and Products 96, 2017, pp. 165-172.
[9] L 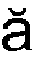 c 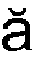 tu 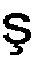 u G.B., Badea N., Ott C., Meghea A.: "Use of various vegetable oils in designing photoprotective nanostructured formulations for UV protection and antioxidant activity" Industrial Crops and Products 67, 2015, pp 18-24.
[10] Chiari B.G., Trovati E., Pecoraro E. et al.: "Synergistic effect of green coffee oil and synthetic sunscreen for health care application" Industrial Crops and Products 52, 2014, pp. 389-393.
[11] Rodrigues F., Alves A.C., Nunes C. et al.: "Permeation of topically applied caffeine from a food by-product in cosmetic formulations: Is nanoscale in vitro approach an option?" International Journal of Pharmaceutics 513 (1-2), 2016, pp. 496-503.
[12] Marto J., Gouveia L.F., Chiari B.G, et al.: "The green generation of sunscreens: Using coffee industrial sub-products" Industrial Crops and Products 80, 2016, pp. 93-100.
[13] Amin R.M., Elfeky S.A., Verwanger T., Krammer B.: "A new biocompatible nanocomposite as a promising constituent of sunscreens" Materials Science and Engineering C, Volume 63, 1 June 2016, Pages 46-51. doi.org/10.1016/j.msec.2016.02.044.
[14] Wongkom L., Jimtaisong A.: "Novel biocomposite of carboxymethyl chitosan and pineapple peel carboxymethylcellulose as sunscreen carrier" Int.J.Biol.Macromolek. Volume 95, February 2017, Pages 873-880.
[15] Morsy R., Ali S.S., EI-Shetehy M.: "Development of hydroxyapatite-chitosan gel sunscreen combating clinical multidrug-resistant bacteria" Journal of Molecular Structure 1143, 2017, pp.251-258.

### Principle of the invention

The aim of the invention is achieved by a UV filter based on polyhydroxybutyrate, which contains particles of polyhydroxybutyrate having a size of 0.2 to 500 µm, which have been functionalized by at least one organic UV filter of natural origin at a total concentration of 2 to 10 %. This UV filter is especially intended for dermal applications for protection against UV radiation. All its ingredients are safe and harmless to human health and the environment.

Depending on the method of preparation, at least one organic UV filter of natural origin, preferably with a possibility of release, is incorporated:
- in the internal structure and/or at the surface of the polyhydroxybutyrate particles, or;
- at the surface of the polyhydroxybutyrate particles and/or in their coating made from liposomes (liposome coating) and/or at the surface of their liposome coating and/or in the space between the polyhydroxybutyrate particles and their liposome coating.

In the case of a UV filter composed of polyhydroxybutyrate particles coated with liposomes, the liposome coating is preferably made from lecithin and reinforced by cholesterol in a preferable ratio of lecithin to cholesterol ranging from 9:1 to 1:1.

Preferably, the concentration of polyhydroxybutyrate in this type of filters is in the range from 20 to 30 %.

The aim of the invention is also achieved by a method of producing a polyhydroxybutyrate-based UV filter in which at least one organic UV filter of natural origin in the form of an extract into distilled water or into a solution of ethanol in distilled water is added to a 0.1 to 20% solution of polyhydroxybutyrate in chloroform, dichloroethane or dichloromethane or in a mixture of at least two of them, and from the thus obtained solution microfibers with a diameter of 0.2 to 500 µm with at least one organic filter incorporated in their structure are obtained by spinning. These microfibers are mechanically disintegrated for further use, for example, by mixing and/or by grinding in a mechanical mill to form polyhydroxybutyrate particles having a size of 0.2 to 500 µm and at least one organic UV filter integrated in their structure at a total concentration of 2 to 10 %. An efficient method for spinning of polyhydroxybutyrate solution is centrifugal spinning.

In another variant, a 0.1 to 20% solution of polyhydroxybutyrate in chloroform, dichloroethane or dichloromethane or in a mixture of at least two of them, is squeezed into a precipitant, e.g., 96% ethanol. This generates the precipitation of the polyhydroxybutyrate microfibers which have a diameter of 0.2 to 500 µm and a porous internal structure. The microfibers formed are then mechanically disintegrated, for example, by mixing and/or by grinding in a mechanical mill to form porous particles of polyhydroxybutyrate having a size of 0.2 to 500 µm. These particles are further incubated for at least 1 hour at room temperature and without access of light with an extract of at least one organic UV filter of natural origin, extracted into a 20-80% solution of ethanol in distilled water. During this incubation, the organic UV filter/filters of natural origin is/are sorbed into the pores of the polyhydroxybutyrate particles and/or on their surface at a total concentration of 2 to 10 %.

Comparable results are also obtained when polyhydroxybutyrate microfibers get into contact with an extract of at least one organic UV filter - extracted into a 20-80% solution of ethanol in distilled water during the mechanical disintegration of the microfibers. Also in this case, the organic UV filter/filters is/are sorbed into the pores of the resulting polyhydroxybutyrate particles and/or on their surface at a total concentration of 2 to 10%.

In another variant of the method of preparation of the UV filter according to the invention, at least one phospholipid, cholesterol and polyhydroxybutyrate are dissolved in chloroform in any order. At least one organic UV filter of natural origin in the form of an extract into distilled water or into a solution of ethanol in distilled water is added to the thus prepared solution. The thus prepared mixture is then diluted with distilled water and exposed to ultrasound, wherein the liposomes formed by the phospholipid/phospholipids bilayer coating with an aqueous phase captured in the internal space are spontaneously sealed around the solid core of polyhydroxybutyrate and the organic UV filter/filters of natural origin, depending on its/their polarity, is/are sorbed in the particles of polyhydroxybutyrate and/or on their surfaces, and/or in their liposome coating and/or at the surfaces of their liposome coating, and/or in the spaces between the polyhydroxybutyrate particles and their liposome coating. This is followed by the removal of chloroform from the mixture thus obtained and by the separation of UV filter particles having a particle size of 0.2 to 100 µm and containing at least one organic UV filter at a total concentration of 2 to 10 %.

In a preferred variant of embodiment, at least one organic UV filter of natural origin is used in the form of an extract into a 30-40% solution of ethanol in distilled water.

To form combined particles consisting of a solid polyhydroxybutyrate core with a liposome coating, the preferably ratio of phospholipid/phospholipids to polyhydroxyalkanoate in a solution is 9:1 to 1:9, and more preferably 8:2 to 7:3.

In a preferred variant of embodiment, lecithin and cholesterol are used as phospholipids in a ratio of 9:1 to 1:1.

### Description of drawings

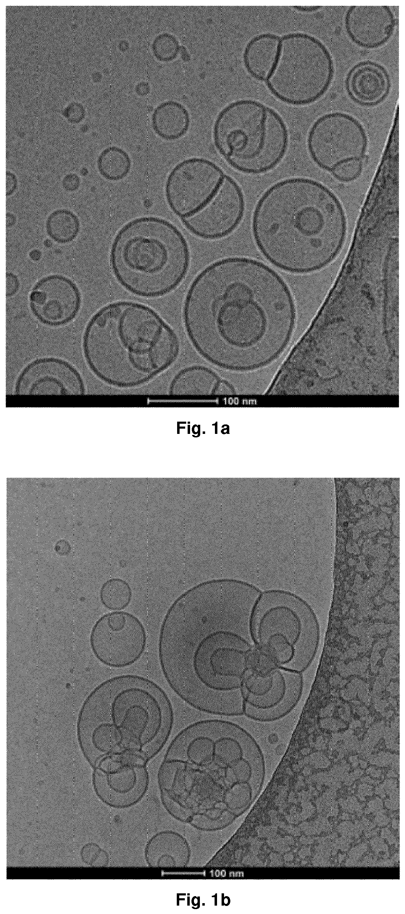

In the accompanying drawings, Fig. 1a shows an image of liposome particles viewed from cryogenic electron microscopy at 50,000 magnification.
Fig. 1b is a cryo-EM image of combined particles consisting of liposomes and PHB with a PHB concentration of 10 % at 80,000 magnification.
Fig. 1c is a cryo-EM image of combined particles consisting of liposomes and PHB with a PHB concentration of 20 % at 50,000 magnification.
Fig. 1d is a cryo-EM image of combined particles consisting of liposomes and PHB with a PHB concentration of 30 % at 80,000 magnification.
Fig. 1e is a cryo-EM image of combined particles consisting of liposomes and PHB with a PHB concentration of 50 % at 10,000 magnification.
Fig. 2 is a graph showing polyphenol content in extracts from green coffee, roasted coffee and coffee bean residues.
Fig. 3 is a graph illustrating the antioxidant activity of extracts from green and roasted coffee, expressed as the equivalent of concentration of Trolox standard.
Fig. 4 is a graph illustrating the efficiency of different types of extracts of green coffee, roasted coffee and coffee bean residues encapsulated into combined particles consisting of liposomes with an addition of 30 % of PHB.
Fig. 5 shows the UV-Vis spectrum of liposomes, PHB and combined particles of liposome and PHB with different concentrations of PHB.
Fig. 6 is a graph depicting the value of the sun protection factor SPF for liposomes, PHB and combined particles of liposome and PHB with different concentrations of PHB.
Fig. 7 is a graph depicting the value of the sun protection factor SPF for liposomes, PHB and combined particles of liposome and PHB with the addition of various amounts of extracts from green and roasted coffee.
Fig.8a is SEM image of PHB microfibers prepared by centrifugal spinning at 1,000 magnification.
Fig. 8b and 8c are SEM images of PHB microfibers prepared by precipitation in a solution at a magnification of 8,000 and 80,000.
Fig. 9a is SEM image of disintegrated PHB microfibers prepared by centrifugal spinning.
Fig. 9b is SEM image of disintegrated PHB microfibers prepared by precipitation in a solution.
Fig. 10a is SEM image of grounded PHB microfibers prepared by centrifugal spinning.
Fig. 10b is SEM image of grounded PHB microfibers prepared by precipitation in a solution.
Fig. 11a shows the UV-Vis spectra of various extracts from green coffee.
Fig. 11b shows the UV-Vis spectra of various extracts from roasted coffee.
Fig. 11c shows a detail of the UV-Vis spectra for the most suitable extracts of green and roasted coffee.
Fig. 12 is a graph showing the cytotoxicity of combined particles composed of liposome and PHB with a PHB concentration of 30 %, expressed as dependence of the keratinocyte viability on the concentration of these particles.

### Examples of embodiment

The UV filter based on polyhydroxybutyrate (PHB) according to the invention consists of PHB particles with a particle size of 0.2 to 500 µm that have been functionalized by at least one known organic UV filter of natural origin at a total concentration of 2 to 10%.

In a preferred variant of embodiment, the core of the UV filter is formed by a particle of PHB, whereby the organic UV filter/filters is/are incorporated into the internal structure of the particle and, optionally, also on its surface. The advantage of this embodiment is that depositing of at least a part of the UV filter/filters in the internal structure of the particles increases its/their stability and their efficiency.

To produce the UV filter according to the invention, three processes can be used. They are based on spinning of 0.1 to 20% solution of PHB in chloroform, dichloroethane or dichloromethane, or a mixture of at least two of them.

In the first variant, the desired organic UV filter/filters, for example, in the form of an extract, is/are added to the spinning solution of PHB and during the subsequent spinning process the UV filter/filters is/are incorporated directly into the structure of the PHB microfibers. Any known manufacturing process can be used for spinning the microfibers - for example, nozzle or nozzle-less electrostatic spinning, precipitation from a solution (e.g., according to the patent CZ 306448) or centrifugal spinning (e.g., according to the patent CZ 2016-423), etc. The result is always PHB microfibers with a diameter of 0.2 to 500 µm depending on the procedure used, with an organic UV filter/filters incorporated in their structure, preferably with a possibility of release. These microfibers are then disintegrated for practical use, for example by mixing and/or mechanical milling or crushing to produce PHB particles of 0.2 to 500 µm in size.

In the second variant, PHB microfibers with a diameter of 0.2 to 500 µm are prepared from the solution of PHB, preferably by precipitation from the solution according to the method of CZ 306448 or by centrifugal spinning according to CZ 2016-423. The PHB microfibers prepared by these processes are naturally porous and have a high specific surface area, whereby, for example, the microfibers prepared by precipitation from the solution according to CZ 306448 have a specific surface area in the range of 36 to 50 m²/g (BET analysis). Porous PHB particles having a particle size of 0.2 to 500 µm are then prepared, for example, by mixing and/or mechanical grinding or crushing. The PHB particles thus prepared are incubated with an extract containing at least one organic UV filter, whereby during the incubation, the organic UV filter is sorbed into the internal structure and at the surface of the particles, preferably in a ratio such that an equivalent of 20 to 100 mg of the original material (e.g. of coffee - see below) is sorbed into their structure per 1 g of fibers (thereby achieving a final concentration of the organic UV filter/filters in these particles of 2 to 10 %). For the incubation, the organic UV filter/filters is/are used in the form of an ethanol extract (extraction into a 20-80% solution of ethanol in distilled water) or an oil extract (extraction into a mixture of methanol and chloroform - Folche extraction). The incubation takes place for at least 1 hour at room temperature without access of light.

In the third variant, at least one organic UV filter is added to the PHB microfibers in the same amount and in the same form as described in the preceding variant already during the disintegration of the microfibers. The disintegration time (typically 5 to 30 minutes) does not have a significant effect on the resulting material and its properties.

In another variant of the UV filter according to the invention, the PHB forms the solid core of the UV filter, the core being provided with a liposome coating, and at least one organic UV filter, depending on its polarity, is arranged in the core or in the liposome coating or in the space between them, which is filled with hydrophilic environment.

To prepare this variant of a UV filter, at first phospholipid/phospholipides (e.g., lecithin) is/are dissolved in chloroform preferably with the addition of cholesterol, which subsequently strengthens the liposome and PHB structure. At least one organic UV filter, e.g., in the form of an extract into distilled water or into a solution of ethanol in distilled water, etc., (see above), is added to the thus prepared solution. The mixture thus formed is then diluted with distilled water and exposed to ultrasound. During this treatment, the solution is homogenized and liposomes formed by phospholipid/phospholipids bilayer coating with an aqueous phase captured in the internal space are spontaneously sealed around the solid core of PHB. Depending on its polarity, the organic UV filter is sorbed into the PHB core, into the liposome coating or in the aqueous phase present between them. After the evaporation of the chloroform, the UV filter particles are isolated from the solution, for example, by centrifugation. These particles are smaller than those prepared by the preceding procedures - their size is approximately from 0.2 to 100 µm. The relative ratio of phospholipid/phospholipids to cholesterol ranges from 9:1 to 1:1.

To prepare the PHB solution of the UV filter according to the invention, any of the known methods can be chosen and especially the one described in CZ 304183 or the analogous international application WO 2014032633, where PHB is produced by bacteria *Cupriavidus necator* H16 on an oil substrate. In this process, a small proportion (about 5 %) of polyhydroxybutyrate valerate (PHBV) is naturally produced. This molecule behaves in the same way as the PHB during the preparation of the UV filter according to the invention and therefore is not specifically mentioned here.

As will be further described in the examples 1 to 4, the UV filters of the present invention achieve, in all variants of embodiment, high sun protection factor (SPF) since, in addition to the organic UV filter/filters, also the PHB (and PHBV) itself contributes to the absorption of the UV radiation, being able to absorb or scatter part of the UV radiation. Depending on its concentration, PHB reaches by itself a protection factor SPF of 5 to 14. Thanks to its hydrophobic character given either by the presence of the liposome coating or by the nature of PHB, the UV filter is sufficiently resistant to water and is also able to partially penetrate into the skin and/or to remain in its upper layers, whereby all its components are safe and harmless to human health and the environment.

### Example 1: Preparation of liposomes, preparation of combined particles of liposomes and PHB and preparation of PHB particles

A solution for preparing liposomes was prepared by dissolving 90 mg of lecithin and 10 mg of cholesterol in chloroform. Subsequently, this solution was diluted with 10 ml of distilled water and then exposed to ultrasound for 1 minute (ultrasonic probe Sonopuls, Bandelin; probe having a diameter of 13 mm; a frequency of 20 kHz). During this treatment, the solution was homogenized and liposomes formed by phospholipids bilayer stabilized by cholesterol with an aqueous phase captured in the internal space were spontaneously sealed. Chloroform was then completely evaporated (residual concentration less than 0.06 pmol/l) from this solution by heating under stirring by a magnetic stirrer and the liposomes formed were separated by centrifugation at 6000 rpm which was carried out for 5 minutes. After the separation of the supernatant, the liposome sediment was resuspended in sterile water.

By the dissolving of 80 mg of lecithin, 10 mg of cholesterol and 10 mg of PHB in chloroform (in any order), a solution was prepared for the production of combined particles of liposome and PHB. This solution was then diluted with 10 ml of distilled water and then exposed to ultrasound (Sonopuls probe ultrasound, Bandelin; probe having a diameter of 13 mm; a frequency of 20 kHz) for 1 minute. In doing so, the solution was homogenized and the liposomes formed by the phospholipids bilayer coating stabilized by cholesterol and with an aqueous phase captured in the internal space were spontaneously sealed around the solid core consisting of a PHB particle having a size up to 100 µm. Subsequently, chloroform was completely evaporated from this solution by heating under constants stirring by magnetic stirrer and the liposomes formed were separated by centrifugation at 6000 rpm which was carried out for 5 minutes. After the separation of the supernatant, the sediment composed of combined particles was resuspended in sterile water. The concentration of PHB in the combined particles thus prepared was 10 %.

Using the he same procedure, combined particles of liposome and PHB consisting of a liposome coating and PHB solid core were prepared, with a concentration of PHB of 20 %, 30 %, 50 % and 70 %. The proportion of PHB always increased at the expense of the proportion of lecithin.

A solution for production of PHB particles was prepared by dissolving 100 mg of PHB in chloroform. This solution was subsequently diluted with 10 ml of distilled water and then exposed to ultrasound for 1 minute (ultrasonic probe Sonopuls, Bandelin; probe having a diameter of 13 mm; a frequency of 20 kHz). In doing so, PHB particles with a diameter of up to 100 µm were spontaneously formed.

In all cases, the PHB prepared according to CZ patent 304183 in the form of a fine powder with a particle size of 0.2 to 10 µm was used.

The unique structure of the particles thus created was verified by cryo-electron microscopy (cryo-TEM, FEI Tecnai F20); their size and stability was assessed by dynamic light scattering (DLS, ZetaSizer Nano ZS, Malvern) - see below.

The PHB commercially available by Biomer company (Germany) was used as a control and comparative sample.

### Determination of the structure of the particles by cryo-electron microscopy

Cryo-electron microscopy is a highly sensitive technique and the only method available today to display the microstructure of particles made up of two different colorless materials. A beam of electrons passes through a very thin layer of the sample, and their interaction is recorded in the form of a detailed structure of the material through which the electrons are passing. Only the samples of liposomes and combined particles with a PHB concentration of up to 50 % were successfully displayed using this method. The samples of combined particles with a PHB concentration of 70 % and 100 % behaved differently already during the preparation and could not be reliably displayed.

Fig.1a is a cryo-electron microscopy image of liposomes without PHB at a magnification 50,000, which shows liposomes mainly in the form of mostly unilamellar particles with a uniform size from about 100 to 300 nm.

Fig. 1b is a cryo-electron microscopy image of combined liposome/PHB particles with a PHB concentration of 10 %, at a magnification of 80,000 in which can be seen a significant effect of PHB on the structure of these particles. The particles formed have a solid PHB core on which the walls of the liposomes are clustered to protect the hydrophobic polymer by their hydrophilic shell.

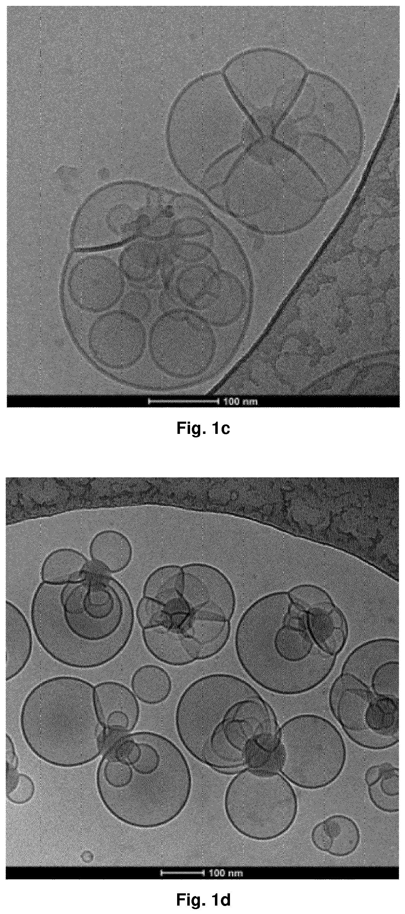

Fig. 1c is a cryo-electron microscopy image of combined liposome/PHB particles with a PHB concentration of 20 %, at a magnification of 50,000, which captures particles similar to those in Fig. 1b, only larger.

Fig.1d is a cryo-electron microscopy image of combined liposome/PHB particles with a PHB concentration of 30 %, at a magnification of 80,000, which shows particles similar to those captured in Figures 1b and 1c but larger. In addition, the particles and clusters of PHB begin to appear between them, which have a higher density than liposomes and their structure changes during longer focused exposure in the microscope.

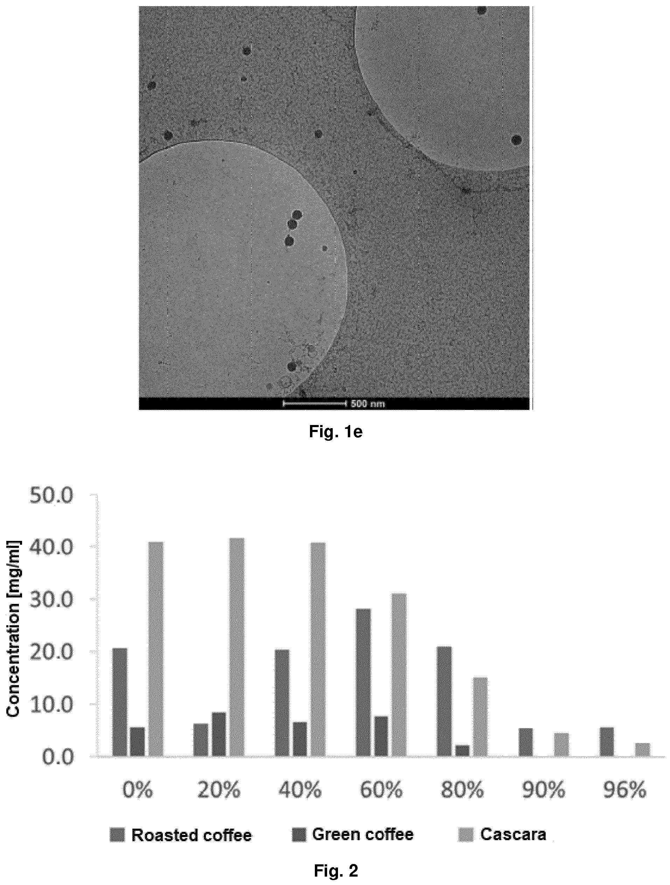

Fig.1e is a cryo-electron microscopy image of combined liposome/PHB particles with a PHB concentration of 50 %, at a magnification of 10,000, showing mainly PHB particles having a size of about 100 nm. Apparently, a liposome monolayer is formed around them to protect them from the hydrophilic aqueous environment.

### Determination of the particle size by DLS, and their colloidal stability

The size of liposomes, PHB particles, and combined liposome/PHB particles with a PHB concentration of 10, 20, 30, 50 and 70 % was determined by the Dynamic Light Scattering (DLS) technique on the ZetaSizer Nano ZS device. The same method was used to determine their polydispersity index (Pdl - particle size scattering) and zeta potential (ZP), which defines their colloidal stability. The values obtained are shown in Table 1.

**Table 1**

| **Sample** | **Particle size [nm]** | **Pdl** | **ZP [mV]** |
|---|---|---|---|
| Liposome particles | 135.9 | 0.207 | -31.6 |
| Combined particles with a PHB concentration of 10% | 194.3 | 0.262 | -36.3 |
| Combined particles with a PHB concentration of 20% | 255.3 | 0.334 | -42.1 |
| Combined particles with a PHB concentration of 30% | 262.2 | 0.301 | -40.0 |
| Combined particles with a PHB concentration of 50% | 218.3 | 0.312 | -53.7 |
| Combined particles with a PHB concentration of 70% | 265.2 | 0.267 | -42.5 |
| PHB particles | 771.9 | 0.739 | -34.4 |

The average particle size is up to 300 nm and the polydispersity index up to 0.32. The sample of PHB particles is the only exception with an average particle size of 770 nm and a Pdl of 0.74. The colloidal stability of all the particles is high and significantly exceeds the limit of stability given by the zeta potential (ZP) -30 mV.

### Example 2: Preparation of liposomes, combined liposome/PHB particles and PHB particles with addition of an organic UV filter of natural origin

### Particles preparation

Liposomes, combined liposome/PHB particles, and PHB particles were prepared by the same methods as described in the Example 1 with the only difference that various extracts of green coffee, roasted coffee or coffee bean residues (cascara) were added to the solutions before sonication, wherein the amount of the extract added was always such that the resulting concentration of the original material was 200 µg/ml, i.e. 2%.

### Extraction and characterization of organic UV filters of natural origin

Active substances extracted from green coffee, roasted coffee and coffee bean residues were used as models of organic UV filter of natural origin. The extraction was carried out for 30 to 60 minutes at room temperature. For hydrophilic components, the extraction agent used was distilled water and 20 to 96% solution of ethanol in distilled water. For the hydrophobic components, the extraction agent used was a mixture of methanol and chloroform in a ratio of 2:1 (Folche extraction). As can be seen below, 20 to 60% solution of ethanol in distilled water was found to be the best extraction agent.

Subsequently, the content of polyphenols, flavonoids and their antioxidant activity was determined with the aid of ABTS radical. In addition, their UV-VIS absorption spectra were measured and the value of the protective factor SPF was calculated from the absorption values measured in the UVB range using Mansur equation.

### Determination of polyphenols

The content of polyphenols in the individual extracts was determined by spectrophotometry. The polyphenol content thus determined is shown graphically in Fig. 2, from which it is clear that the highest content of polyphenols from green coffee was extracted into a 40% solution of ethanol in distilled water; from the roasted coffee into a 60% solution of ethanol in distilled water and from the residues of coffee beans into a 20% solution of ethanol in distilled water. At the same time, it is evident from Fig. 2 that the extraction is most effective in the case of green coffee when using solutions of 20 % and 40 % of ethanol in distilled water, in the case of roasted coffee when using a 60% solution of ethanol in distilled water.

The Folche extraction was performed only on a sample of roasted coffee, where the content of polyphenols coincided with the extract prepared using a 90% solution of ethanol in distilled water.

### Determination of flavonoids

At the same time, the content of flavonoids in the individual extracts was determined by spectrophotometry. Extraction into distilled water or into a 20 to 80% solution of ethanol in distilled water resulted in a higher flavonoid content in green coffee samples, extraction into a 90 and 96% solution of ethanol resulted in a higher flanovoid content in roasted coffee samples. Overall, however, the flavonoid content in roasted coffee extract was lower than that of extract from the green coffee and ranged from 5 to 35 mg/ml; for the extract from the green coffee, the flavonoids content was by 10 to 15 % higher. On the contrary, with Folche extraction, the flavonoid content extracted from the roasted coffee was up to four times higher - 165 mg/ml.

### Antioxidant activity

The antioxidant activity of green coffee extracts extracted into distilled water and into 20 to 60% solutions of ethanol in distilled water was very similar, ranging from 5 to 7 mg.g⁻¹ (expressed as mg of Trolox equivalent per gram of initial material of natural UV filter). Similar antioxidant activity values were also measured for roasted coffee extracts extracted into 20, 90 and 96% solutions of ethanol in distilled water. When extracting green coffee samples into solutions with higher ethanol concentrations, the antioxidant activity of the samples was almost zero. On the contrary, when extracting roasted coffee samples into 40 to 80 % solutions of ethanol into distilled water, the antioxidant activity was significantly higher and values between 20 and 30 mg.g⁻¹ were obtained.

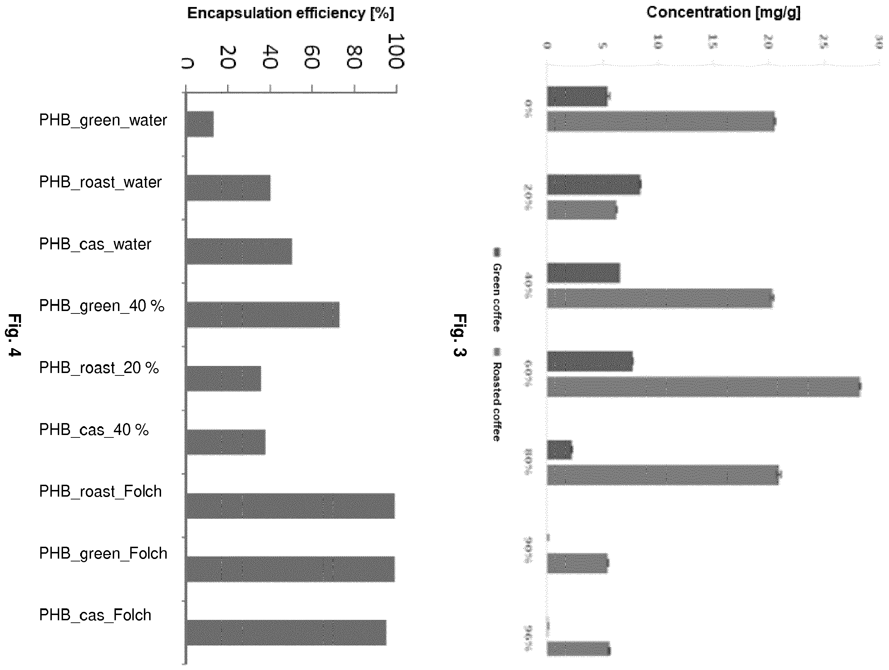

Fig. 3 is a graph showing the antioxidant activity of the individual extracts from green and roasted coffee extracted into distilled water (in Fig. 3, described as 0%) and into 20, 40, 60, 80, 90 and 96% solutions of ethanol in distilled water.

### Encapsulation efficiency

By determining the amount of polyphenols released from the particles, the efficiency of encapsulation of various types of extracts was determined for combined particles with PHB concentration of 30 %. Fig. 4 illustrates the efficiency of encapsulation thus obtained in a graph. The individual columns represent, from the left: the encapsulation efficiency of the combined particles for the extract from green coffee, extracted into distilled water; the encapsulation efficiency of the combined particles for the extract from roasted coffee, extracted into distilled water; the encapsulation efficiency of the combined particles for the extract from coffee bean residues extracted into distilled water; the encapsulation efficiency of the combined particles for the extract from green coffee, extracted into a 40% solution of ethanol in distilled water; the encapsulation efficiency of the combined particles for the extract from roasted coffee, extracted into a 20% solution of ethanol in distilled water; the encapsulation efficiency of the combined particles for the extract from coffee bean residues, extracted into a 40% solution of ethanol in distilled water; the encapsulation efficiency of the combined particles for the extract from roasted coffee, extracted by Folch method; the encapsulation efficiency of the combined particles for the extract from green coffee, extracted by Folch method; the encapsulation efficiency of the combined particles for the extract from coffee bean residues, extracted by Folch method.

### Determination of the protective factor SPF of liposomes, combined liposome/PHB particles and PHB particles

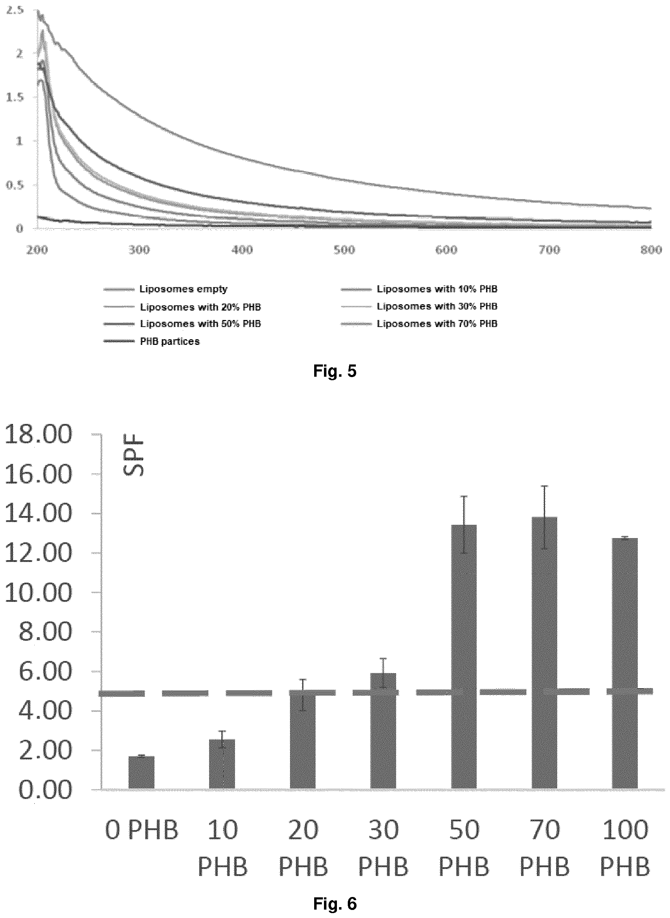

Liposomes, PHB particles and combined particles composed of liposomes and PHB with a PHB concentration of 10, 20, 30, 50 and 70 % were diluted in ethanol and then their absorbance was measured on a UV-Vis spectrophotometer for the radiation with a wavelength of 280 to 320 nm (with a step of 5 nm). Illustrative UV-Vis spectra of these particles are shown in Fig. 5. The Mansur equation was then used to determine the sunscreen protection factor SPF of the particles from these spectra. From the results illustrated in Fig. 6 it is clear that the highest values of the protective factor SPF were achieved by the combined liposome/PHB particles with a PHB concentration of 70 %. However, it is difficult to incorporate the organic UV filter into these particles because they are already too compact and because they are made up mainly of PHB coated with a single layer of phospholipids (see, for example, Fig. 1e). In terms of particle structure, stability and combined effect of liposomes, PHB and organic UV filter, the combined particles with a PHB concentration of about 20 to30 % appear to be the most appropriate for dermal application, these particles having a SPF of about 6 and not being cytotoxic (see Example 4).

### Determination of the protective factor SPF of extracts, liposomes, combined liposome/PHB particles, and PHB particles containing an organic UV filter

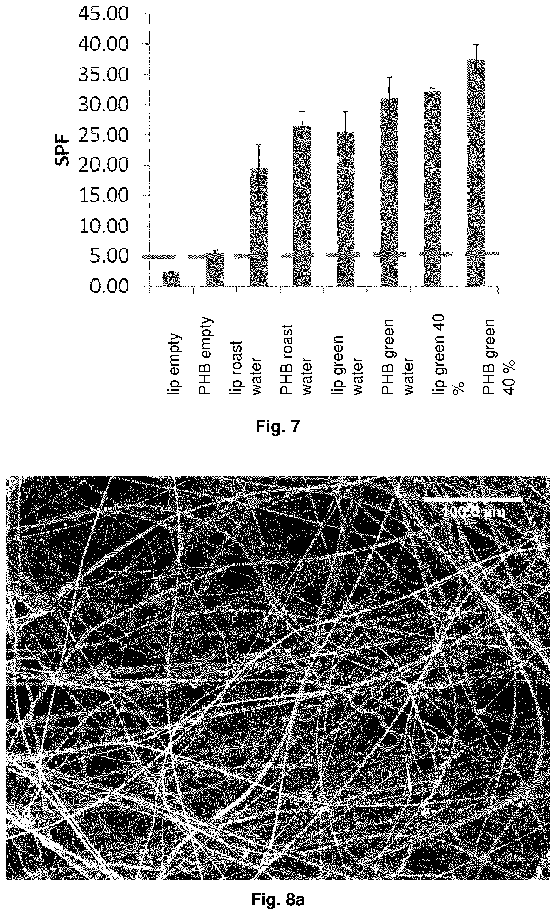

Liposomes and PHB particles without extract (Fig. 7 - empty liposomes, or empty PHB), liposomes and PHB particles containing an extract from roasted coffee, extracted into distilled water (Fig. 7 - lipid in water, or PHB in water), liposomes and PHB particles containing an extract from green coffee, extracted into distilled water (Fig. 7 - lipid green water, or PHB green water), liposomes and PHB particles containing an extract from green coffee, extracted into a 40 % solution of ethanol in distilled water (Fig. 7 - lip green 40 %, or PHB green 40 %) were diluted with ethanol and their absorbance was measured for wavelengths from 280 to 320 nm (with a step of 5 nm) by UV-Vis spectrophotometer. Subsequently, from these spectra, the SPF factor, which is shown graphically in Figure 7, was calculated by the Mansur equation, the Figure 7 showing that the highest value of SPF was obtained for the sample containing an extract from green coffee extracted into 40% solution of ethanol in distilled water. Its SPF protection factor was 31.8. The high antioxidant effect of the extracts used also contributes significantly to the protective effect against UV damage.

Next, liposomes, PHB particles, and combined liposome/PHB particles with a PHB concentration of 10, 20, 30, 50 and 70 %, containing an extract from green coffee (extracted into a 40% solution of ethanol in distilled water) at a final concentration of 200 µg/ml, i.e. 2 %, were prepared. The coffee extract was partly incorporated into the PHB core, partly incorporated into the liposome coating and partly incorporated into the space between the core and the coating, filled with hydrophilic environment. The values of the SPF protection factor of these particles ranged from 25.75 to 38.5 - see Table 2.

**Table 2**

| **Particles** | **SPF** | **Standard deviation SD** |
|---|---|---|
| Liposomes without extract | 3.95 | 0.32 |
| Liposomes with extract from green coffee | 33.80 | 4.12 |
| Combined particles having PHB concentration of 10 % with extract from green coffee | 34.75 | 2.56 |
| Combined particles having PHB concentration of 20 % with extract from green coffee | 32.62 | 1.88 |
| Combined particles having PHB concentration of 30 % with extract from green coffee | 38.50 | 2.54 |
| Combined particles having PHB concentration of 50 % with extract from green coffee | 25.75 | 3.80 |
| Combined particles having PHB concentration of 70 % with extract from green coffee | 27.80 | 4.60 |
| PHB particles with extract from green coffee | 32.20 | 3.64 |
| Extract from green coffee, extracted into solution of 40 % of ethanol in distilled water | 31.50 | 0.59 |

### Example 3: PHB particles generated by disintegration of PHB microfibers material

PHB, having an average molecular weight of 50,000 to 100,000 Da, was prepared by the method described in the CZ patent 304183 and ground into granulates having a particle size of 200 to 500 µm. The granulate thus formed was then dissolved in chloroform, dichloroethane or dichloromethane, or in a mixture of at least two of them to prepare PHB solutions with a PHB concentration ranging from 0.1 to 20 % by weight. These solutions were then spun by any spinning method, for example, by nozzle or nozzle-less electrostatic spinning, preferably by precipitation in a solution (e.g., according to CZ 306448) or centrifugal spinning (e.g., according to CZ 2016-423) etc., thereby forming PHB microfibers with a diameter between 0.2 and 500 µm.

### Preparation of porous microparticles from PHB microfibers

A porous PHB microfiber with a diameter of 0.5 to 100 µm was prepared by centrifugal spinning (according to CZ PV 2016-423) from a 4% solution of PHB in chloroform, as described above. The clear, slightly yellow PHB solution was fed at a rate of 1 ml/s to a rotating disc, which was rotated at 20,000 rpm. In doing so, a network of white fibers was immediately formed at a distance of about 10 cm from the disc. These microfibers were then dried and characterized using electron microscopy. SEM image of microfibers thus prepared at a magnification of 1,000 is shown in Fig. 8a.

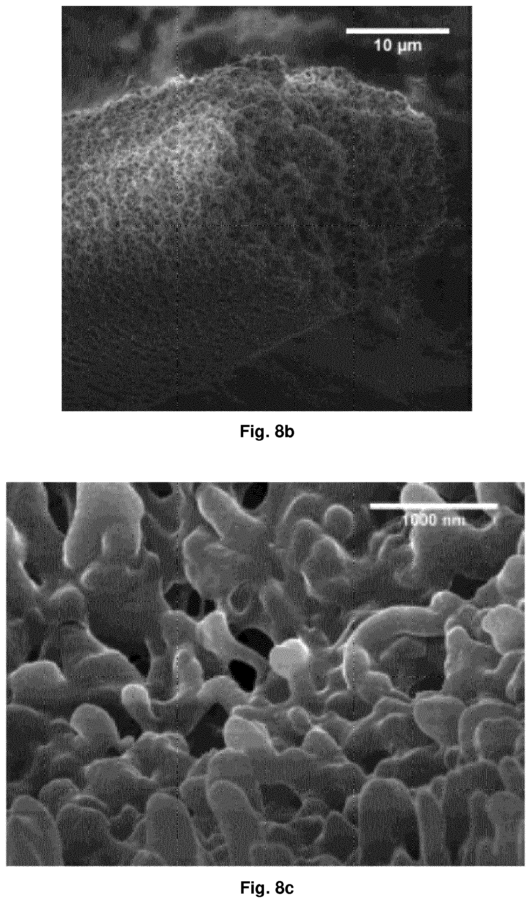

PHB microfibers with higher porosity are prepared by precipitation from a solution according to CZ 306448. PHB is dissolved in chloroform to prepare a solution with a PHB concentration ranging from 0.1 to 20 %. This solution is then extruded into a suitable precipitant, such as ethanol of 96% concentration. The diameter of the microfibers thus created is about 0.2 to 100 µm and their length ranges from hundreds of micrometers to millimeters. SEM images of the microfibers thus prepared at a magnification of 8,000 are shown in Fig. 8b and at a magnification of 80,000 in Fig. 8c.

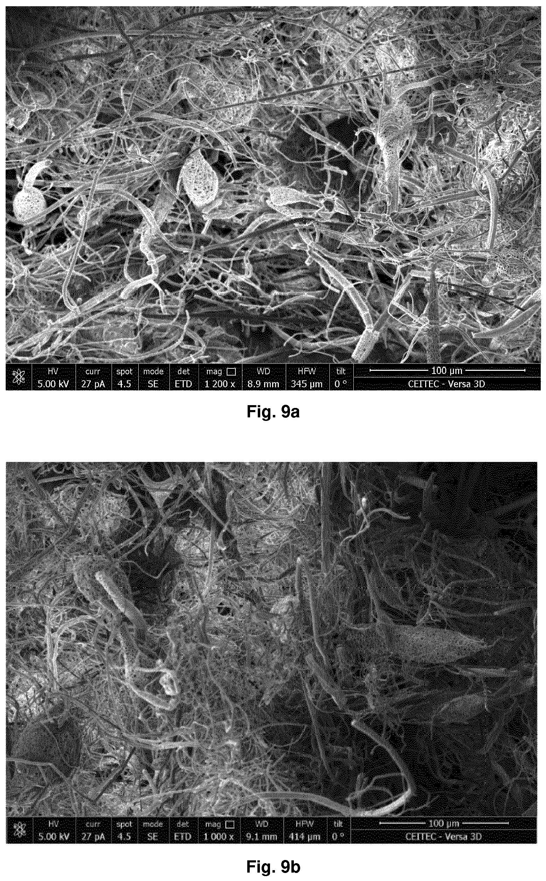

The microfibers produced by any of these methods were cut and mixed into particles of a size in the range of 0.2 to 500 µm - see Fig. 9a, which is SEM image of cut and mixed microfibers prepared by centrifugal spinning and Fig. 9b, which is SEM image of cut and mixed microfibers prepared by extrusion into a precipitant. If cutting and mixing is not sufficient, it can be replaced or supplemented by grinding in a mechanical mill.

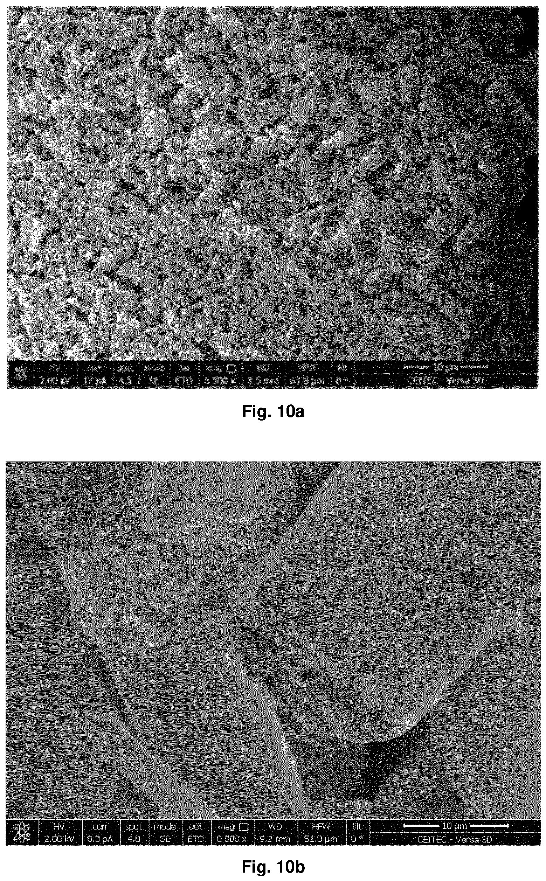

Fig. 10a is SEM image of PHB particles thus prepared having a dimension of 1 µm in each direction.

The PHB particles thus prepared are similar in size to the ground commercial polymer (Biomer) but have a significantly higher sorption capacity for natural substances.

All particles prepared by disintegration of PHB microfibers can be used as carriers of organic UV filters of natural origin for dermal applications (in sunscreens or facial masks, etc.).

### Example 4: Method of functionalization of PHB particles produced by disintegration of PHB microfibers with a natural UV filter

The possibility of sorption of natural UV filters into the structure of microfibers during their formation by spinning or into the microfibers already produced was examined for the PHB particles prepared according to the procedure described in Example 3, which was then evaluated qualitatively using the fluorescence lifetime imaging microscopy (FLIM). Sorption occurring during the spinning resulted in the incorporation of an organic UV filter into the fiber structure, sorption during the disintegration was initiated by the addition of the UV filter just before the disintegration. The sorption to the prepared fibers was carried out for 1 hour at room temperature without access of light.

Since the effect of the disintegration of PHB microfibers on their protection factor SPF has not yet been described, not only disintegrated PHB microfibers have been tested but also fibers containing various coffee extracts added to the microfibers at different stages of their processing (during their preparation, during their disintegration, after their disintegration).

Several materials, prepared by different procedures and under different conditions, were carefully tested - see Table 3. Ground PHB commercially available from Biomer company and supernatant collected from this sample were used as the control.

### UV-VIS spectra of active substances

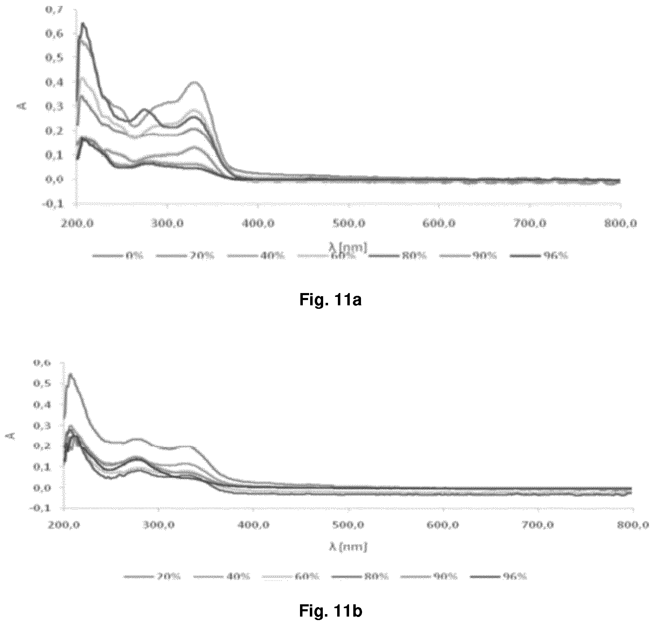

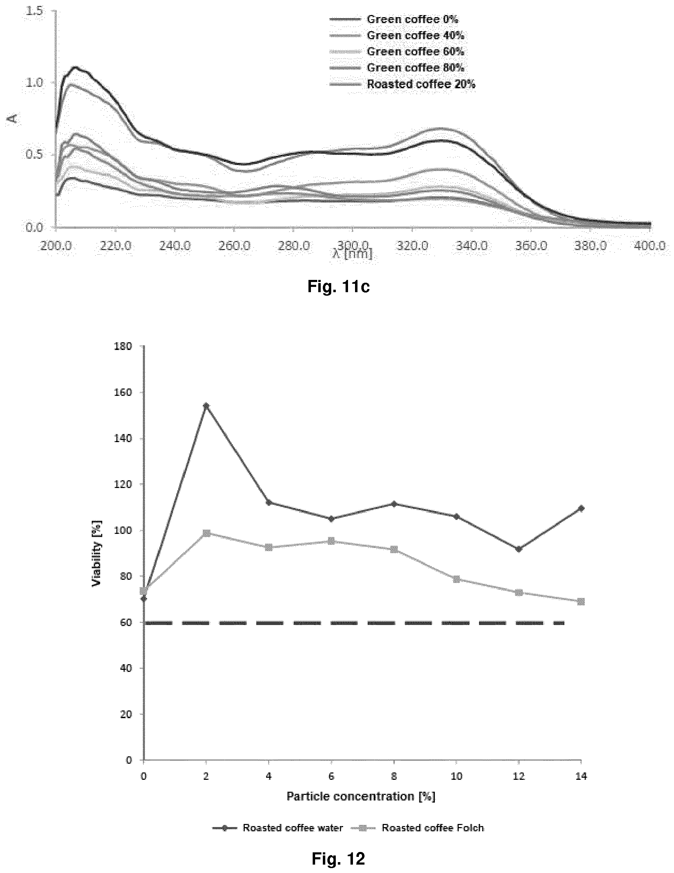

The absorption for radiation with a wavelength of 280 nm to 800 nm (with a step of 5 nm) was measured by a UV-VIS spectrophotometer for samples of extracts from green coffee (absorption spectra shown in Fig. 11a) and from roasted coffee (absorption spectra shown in Fig. 11b), extracted into distilled water, into a 20% solution of ethanol in distilled water, a 40% solution of ethanol in distilled water, 60% solution of ethanol in distilled water, a 80% solution of ethanol in distilled water, a 90% solution of ethanol in distilled water and a 96% solution of ethanol in distilled water (Fig. 11c shows an appropriate portion of the absorption spectrum of the selected samples). The most appropriate extracts for preparing the UV filters according to the invention are samples of green coffee extracted into distilled water, into a 40% solution of ethanol in distilled water, a 60% solution of ethanol in distilled water and 80% solution of ethanol in distilled water, and the extract of roasted coffee, extracted into a 20% solution of ethanol in distilled water - see Fig. 11c.

On the basis of the thus obtained spectra and using the Mansur equation, the value of the protection factor SPF was calculated for the PHB microfibres without any extract and the particles prepared by disintegration of these microfibers without any extract content and for the PHB microfibers and the particles prepared by disintegration of these microfibers with the extract of green coffee, extracted into 40% solution of ethanol in distilled water and the particles prepared by disintegration of these microfibers - see Table 3.

**Table 3**

| **Sample** | **PHB** | | **PHB with extract from green coffee** | |
|---|---|---|---|---|
| | **SPF** | **SD** | **SPF** | **SD** |
| Disintegrated PHB microfibers (9 g of microfibers/200 ml of distilled water, mixed) | 3.95 | 0.32 | 12.44 | 1.20 |
| Particles of PHB obtained by a method of CZ 304183 | 12.2 | 0.82 | 35.15 | 2.56 |
| Disintegrated PHB microfibers (4.5 g of microfibers/200 ml of distilled water, mixed for 10 minutes) | 3.75 | 0.54 | 15.85 | 1.98 |
| Disintegrated PHB microfibers (4.5 g of microfibers/200 ml of distilled water, mixed for 5 minutes) | 3.62 | 0.46 | 17.75 | 3.53 |
| PHB microfibers (4.5 g of microfibers/200 ml of distilled water) prepared by centrifugal spinning with addition of 10 % of coffee extract | - | - | 15.85 | 1.98 |
| PHB microfibers (4.5 g of microfibers/200 ml of distilled water) prepared by precipitation of PHB solution in a precipitant, disintegrated in the presence of 15 ml of extract from green and roasted coffee extracted into 40% solution of ethanol in distilled water | - | - | 17.75 | 3.53 |
| Biomer - control sample. PHB particles of size in the range of 1 to 100 µm | 6.42 | 0.72 | 18.50 | 2,82 |
| Supernatant of the control sample | 5.82 | 0.28 | - | - |
| PHB particles with size up to 200 µm, prepared by disintegration of microfibers prepared by precipitation of a PHB solution | 12.46 | 0.59 | 36.20 | 4,41 |
| PHB particles with size up to 200 µm, prepared by disintegration of microfibers prepared by centrifugal spinning of a PHB solution | 11.65 | 0.68 | 34,50 | 3,82 |

The sample of ground PHB prepared by the procedure according CZ 304183 had a protective factor SPF 12.2, whereas the PHB of the Biomer company in the form of particles having a size varying from 1 to 100 µm had a SPF of 6.4. The protective factor SPF of the PHB particles prepared by coarse disintegration of PHB microfibers prepared by centrifugal spinning (Fig. 9a) was 3.75 (when mixed for 10 minutes), or 3.62 (when mixed for 5 minutes). From this it is clear that the disintegration time did not have a significant effect on the final value of the SPF. The protection factor SPF of PHB particles prepared by gentle disintegration of PHB microfibers prepared by centrifugal spinning (Fig. 10a) was 11.65; the SPF of the PHB particles prepared by precipitation of the solution was 12.46. As can be seen in Fig. 10b, even after disintegration, the porous PHB microfibers have preserved their inner complex nanostructure, which is very important for the sorption of organic UV filters.

In addition, it is evident from Table 3 that after the addition of the extract from green coffee, extracted into a 40% solution of ethanol in distilled water, there was a physical adsorption of this material into the PHB microfibers, whereby the protection factor SPF was increased by 7 up to 23 in the majority of the samples. Overall, it was possible to achieve a SPF of up to 35, which is similar to the SPF obtained for combined liposome/PHB particles containing an extract from green coffee with the same concentration (compare Fig. 7).

### Determination of interaction of components of the UV filters according to the invention with cells

### Tests of cytotoxicity and genotoxicity

### Cytotoxicity

The cytotoxicity of the extracts and the liposomes was determined by the MTT assay, which is based on the fact that the yellow dye MTT (3-[4,5-dimethylthiazol-2-yl]-2,5-diphenyltetrazolium bromide) is reduced by mitochondrial enzymes of the cells respiratory chain to a purple formazan derivative that remains within the cells in the form of insoluble granules. By adding the SDS detergent (sodium dodecyl sulfate), the dye is released from the cells and dissolved to create a clear purple solution which is measured in a spectrophotometer at 540 nm. The test is always performed with concentration series of the material tested. In this case, liposomes containing 30 % of PHB at a final concentration of 2 to 14 % of the total volume were added to human cells in the medium and the mixture thus created was incubated for 24 hours. In doing so, it was found out that neither the liposomes nor the combined particles consisting of liposomes and PHB exhibited any cytotoxic effect.

Fig. 12 shows a graph representing the keratinocyte viability depending on the concentration of combined liposome/PHB particles having a PHB concentration of 30 % and containing an extract from green or roasted coffee at a concentration of 200 µg/g. The value of 60 % of less of surviving cells is considered to be the threshold value for determining a sample as cytotoxic. The Table 4 summarizes the results of the intensity of the purple solution of dissolved formazan crystals, determined by spectrophotometry, expressing the amount of living cells of the blank (%) compared to the intensity of this solution depending on the amount of the combined particles. The more combined particles were added, the lower cell viability was obtained. The concentration of the combined particles higher than 12 % can be considered as cytotoxic. The addition of fibers did not exhibit any cytotoxicity.

**Table 4**

| **Material** | **Viability %** | **Standard deviation SD** |
|---|---|---|
| Combined particles with coffee extract, concentration of 2 % | 98 | 4.2 |
| Combined particles with coffee extract, concentration of 4 % | 94 | 8.3 |
| Combined particles with coffee extract, concentration of 6 % | 84 | 7.2 |
| Combined particles with coffee extract, concentration of 8 % | 75 | 5.6 |
| Combined particles with coffee extract, concentration of 10 % | 66 | 6.8 |
| Combined particles with coffee extract, concentration of 12 % | 61 | 5.2 |
| Combined particles with coffee extract, concentration of 14 % | 55 | 7.4 |
| 2.2 mg of ground PHB in a size up to 500 µm prepared according to the CZ patent 304183 | 82 | 8.8 |
| 2.2 mg of ground PHB microfibers, prepared by precipitation of PHB solution | 83.5 | 9.6 |
| 2.2 milligrams of PHB microfibers ground, prepared by centrifugal spinning of a PHB solution | 87.5 | 7.6 |
| 3 mg of ground PHB up to 500 µm, prepared according to CZ patent 304183 | 72.2 | 6.5 |
| 3 mg of ground PHB microfibers, prepared by precipitation of PHB solution | 62.8 | 6.3 |
| 3 mg PHB of ground microfibers, prepared by centrifugal spinning of PHB solution | 65.2 | 8.2 |

### Genotoxicity

In the same samples, their genotoxicity was determined by the SOS chromotest. This method is based on monitoring the expression of genes induced by toxic agents in response to DNA damage. The tested sample acted during incubation on a genetically modified E. *coli* strain, in which the β-galactosidase gene is controlled by the same promoter as a gene involved in reparation systems. After a defined time, the galactosidase expression of the exposed cultures corresponding to the SOS expression rate is evaluated. The expression rate is measured by spectrophotometry. A substance is considered as genotoxic when its SOSIF value is greater than or equal to 1.5. None of the tested materials has reached this value.

### Example 5: A method of functionalizing PHB particles formed by disintegration of PHB microfibers by natural UV filter

For PHB particles prepared according to the process of Example 3, the possibility of sorption of other natural UV filters was examined. For this purpose, a carrot seed extract into a 40% solution of ethanol in distilled water was used.

From the absorbance for the radiation with a wavelength of 280 to 800 nm measured on the UV-VIS spectrometer, the SPF value was determined using the Mansur equation for a new sample of ground PHB and for a sample of ground PHB with the carrot seed extract - see Table 5.

**Table 5**

| **Sample** | **PHB** | | **PHB with carrot seed extract** | |
|---|---|---|---|---|
| | **SPF** | **SD** | **SPF** | **SD** |
| PHB particles having a size of up to 200 µm prepared by disintegration of microfibers obtained by precipitation of PHB solution | 12.46 | 0.59 | 31.21 | 3.20 |

The sample of ground PHB prepared according to the procedure of CZ patent 304183 had a protective factor SPF 12.46, whereas after the addition of the carrot seed extract extracted into 40% solution of ethanol in distilled water and after 1 hour incubation, physical adsorption of this material to the PHB microfibers occurred, wherein the value of the SPF factor increased by 18.75 units. Overall, values of SPF of about up to 31 were obtained, which are similar values to the values as in the case of the combined liposome/PHB particles with the same concentration of green coffee extract - see Table 2.

### Example 6: A method of functionalizing PHB particles formed by disintegration of PHB microfibers by natural UV filter

For PHB particles prepared according to the procedure of Example 3, the possibility of sorption of other natural UV filters was examined. For this purpose, an extract of Indian beech fruit (caranja) in a 40% solution of ethanol in distilled water was used.

From the absorbance for radiation with a wavelength of 280 to 800 nm measured on the UV-VIS spectrometer, the SPF factor was determined using the Mansur equation for a new sample of ground PHB and for a sample of ground PHB with Indian beech fruit extract - see Table 6.

**Table 6**

| **Sample** | **PHB** | | **PHB with Indian beech fruit extract** | |
|---|---|---|---|---|
| | **SPF** | **SD** | **SPF** | **SD** |
| PHB particles having a size of up to 200 µm prepared by disintegration of PHB microfibers obtained by precipitation from PHB solution | 12.46 | 0.59 | 33.13 | 3.38 |

A sample of ground PHB prepared according to the procedure of CZ patent 304183 had a protective factor SPF 12.46, whereas after the addition of Indian beech fruit extract extracted into 40% solution of ethanol in distilled water and after 1 hour incubation, physical adsorption of this material to PHB microfibers occurred, wherein the value of the SPF factor increased by 20.67 units. Overall, SPF values of about 33 were obtained, which are similar values to the values as in the case of combined liposome/PHB particles with green coffee extract into a 20% solution of ethanol in distilled water - see Table 2.

### Example 7: A method of functionalizing PHB particles formed by disintegration of PHB microfibers by natural UV filter

For PHB particles prepared according to the procedure of Example 3, the possibility of sorption of other natural UV filters was examined. Extracts from the fruits of selected vegetables (tomato, paprika, carrot, spinach leaves) were used for this purpose - separately or in various combinations. The same ratio was always used: 1 g of natural material extracted into 2 ml of a 40% solution of ethanol in distilled water.

From the absorbance for radiation with a wavelength of 280 to 800 nm measured on the UV-VIS spectrometer, the value of the protective factor SPF was subsequently calculated by the application of the Mansur equation for a new sample of ground PHB and for a sample of ground PHB with the extract from the fruits of individual vegetables and their mixtures - see Table 7.

**Table 7**

| **Sample** | **Type of extract** | **PHB** | | **PHB with vegetable extract** | |
|---|---|---|---|---|---|
| | | **SPF** | **SD** | **SPF** | **SD** |
| PHB particles having a size of up to 200 µm prepared by disintegration of PHB microfibers obtained by precipitation from PHB solution | | 12.46 | 0.59 | - | - |
| PHB particles 200 µm | tomato | | | 14.58 | 0.68 |
| PHB particles 200 µm | carrot | | | 16.30 | 0.90 |
| PHB particles 200 µm | paprika | | | 15.95 | 0.92 |
| PHB particles 200 µm | spinach | | | 18.24 | 0.88 |
| PHB particles 200 µm | spinach + carrot | | | 33.06 | 2.24 |
| PHB particles 200 µm | paprika + spinach | | | 32.64 | 3.06 |

A sample of ground PHB prepared according to the procedure of CZ patent 304183 had a protective factor SPF 12.46. After the addition of individual kinds of vegetables and their mixtures extracted into a 40 % solution of ethanolin distilled water and after 1 hour incubation, physical adsorption of this material on PHB microfibers occurred. The value of the SPF factor increased by 2.2 to 5.8 units for the individual extracts, and up to 20 units for a combination of two different extracts. In the case of combined extracts, the same amount of each of the extracts was used, i.e. a total of twice the amount of extract to be sorbed. The cumulative effect of spinach extracts with either paprika or carrot extract was observed. Overall, values of SPF of about 32 - 33 were obtained, similar to the values as in the case of combined liposome/PHB particles with green coffee extract extracted into a 20% solution of ethanol in distilled water - see Table 2.

## Claims

**1.** A UV filter based on polyhydroxybutyrate, **characterized in that** it contains polyhydroxybutyrate particles, ranging in size from 0.2 to 500 µm, which are functionalized by at least one organic UV filter of natural origin at a total concentration between 2 and 10 %.

**2.** The UV filter according to claim 1, **characterized in that** at least one organic UV filter of natural origin is incorporated in the inner structure and/or at the surface of the polyhydroxybutyrate particles.

**3.** The UV filter according to claim 2, **characterized in that** at least one organic UV filter of natural origin is incorporated in the inner structure and/or at the surface of the polyhydroxybutyrate particles with the possibility of release.

**4.** The UV filter according to claim 1 or 2, **characterized in that** the polyhydroxybutyrate particles are porous and at least one organic UV filter of natural origin is incorporated into their pores and/or on their surface.

**5.** The UV filter according to claim 4, **characterized in that** at least one organic UV filter of natural origin is incorporated in the pores and/or at the surface of the polyhydroxybutyrate particles with the possibility of release.

**6.** The UV filter according to claim 1, **characterized in that** the polyhydroxybutyrate particles have a size of 0.2 to 100 µm and are coated with a liposome coating, whereby the spaces between the polyhydroxybutyrate particles and their liposome coating are filled with a hydrophilic environment, and at least one organic UV filter of natural origin is deposited at the surfaces of the polyhydroxybutyrate particles and/or in their liposome coatings and/or at the surfaces of their liposome coatings and/or in the spaces between the polyhydroxybutyrate particles and their liposome coatings.

**7.** The UV filter according to claim 6, **characterized in that** at least one organic UV filter of natural origin is deposited at the surfaces of the polyhydroxybutyrate particles and/or in their liposome coatings and/or at the surfaces of their liposome coatings and/or in the spaces between the polyhydroxybutyrate particles and their liposome coatings with the possibility of release.

**8.** The UV filter according to claim 6 or 7, **characterized in that** the liposome coating of the polyhydroxybutyrate particles consists of lecithin and cholesterol in a ratio of 9:1 up to 1:1.

**9.** The UV filter according to any of claims 6 to 8, **characterized in that** the concentration of polyhydroxybutyrate in the UV filter particles is 20 to 30 %.

**10.** A method of production of a UV filter based on polyhydroxybutyrate, **characterized in that** at least one organic UV filter of natural origin in the form of an extract into distilled water or into a solution of ethanol in distilled water is added to 0.1 to 20% solution of polyhydroxybutyrate in chloroform, dichlorethane or dichlormethane, or a mixture of at least two of them, and from thus obtained solution, microfibers with diameter of 0.2 to 500 µm with at least one UV filter incorporated into their structure are prepared by spinning whereupon the microfibers are mechanically disintegrated to form polyhydroxybutyrate particles having a size of 0.2 to 500 µm, in the structure of which at least one organic UV filter is incorporated at a total concentration of 2 to 10 %.

**11.** The method according to claim 10, **characterized in that** the polyhydroxybutyrate microfibers are formed by centrifugal spinning.

**12.** The method according to claim 10, **characterized in that** the polyhydroxybutyrate microfibers with at least one organic UV filter incorporated in their structure are mechanically disintegrated by mixing and/or grinding in a mechanical mill.

**13.** A method for production of a UV filter based on polyhydroxybutyrate, **characterized in that** 0.1 to 20% solution of polyhydroxybutyrate in chloroform, dichlorethane or dichlormethane, or a mixture of at least two of them, is extruded into a precipitator, whereby polyhydroxybutyrate microfibers having a diameter of 0.2 to 500 µm and porous internal structure are precipitated, subsequently the thus formed polyhydroxybutyrate microfibers are mechanically disintegrated to form porous polyhydroxybutyrate particles having a size of 0.2 to 500 µm, which are incubated for at least 1 hour at room temperature and without access of light, with an extract of at least one organic UV filter of natural origin extracted into a 20 to 80 % solution of ethanol in distilled water, wherein the organic UV filter of natural origin is sorbed at a total concentration of 2 to 10% in the pores of the polyhydroxybutyrate particles and/or on their surface.

**14.** The method according to claim 13, **characterized in that** the solution of polyhydroxybutyrate in chloroform, dichloroethane or dichloromethane or a mixture of at least two of them is precipitated with 96% ethanol.

**15.** The method according to claim 13, **characterized in that** the polyhydroxybutyrate microfibers with at least one organic UV filer incorporated in their structure are mechanically disintegrated by mixing and/or grinding in a mechanical mill.

**16.** The method for production of a UV filter based on polyhydroxybutyrate, **characterized in that** 0,1 to 20% solution of polyhydroxybutyrate in chloroform, dichlorethane or dichlormethane, or a mixture of at least two of them, is extruded into a precipitator, whereby polyhydroxybutyrate microfibers having a diameter of 0.2 to 500 µm and a porous internal structure are precipitated, then the thus formed polyhydroxybutyrate microfibers are disintegrated in a presence of an extract of at least one organic UV filter extracted into 20 to 80% solution of ethanol in distilled water, while during their disintegration the organic UV filter/filters is/are sorbed at a total concentration of 2 to 10% into the pores and/or at the surface of the polyhydroxybutyrate particles being formed.

**17.** The method according to claim 16, **characterized in that** the polyhydroxybutyrate microfibers are mechanically disintegrated by mixing and/ or grinding in a mechanical mill.

**18.** The method for production of a UV filter based on polyhydroxybutyrate, **characterized in that** at least one phospholipid, cholesterol and polyhydroxybutyrate are dissolved in chloroform in any order, and at least one organic UV filter of natural origin in a form of extract into distilled water or solution of ethanol in distilled water is added into the thus obtained solution, then the thus formed mixture is diluted with distilled water and is exposed to ultrasound, while liposomes formed of double-layer phospholipid/phospholipids with an internal space filled with aqueous phase spontaneously seal around a solid polyhydroxybutyrate core and an organic UV filter/filters of natural origin deposits/deposit in the polyhydroxybutyrate particles and/or on their surfaces and/or in their liposome coatings and/or the surfaces of their liposome coatings and/or in the spaces between the polyhydroxybutyrate particles and their liposome coatings, then the chloroform is removed from the mixture and the UV filter particles which have a size of 0.2 to 100 µm are separated therefrom and which contain at least one organic UV filter at a total concentration of 2 to 10 %.

**19.** The method of production according to claim 18, **characterized in that** at least one organic UV filter of natural origin is added in the form of an extract into a 30 to 40 % solution of ethanol in distilled water.

**20.** The method of production according to claim 18 or 19, **characterized in that** the ratio between phospholipid and polyhydroxybutyrate in the solution ranges from 9:1 up to 1:9.

**21.** The method of production according to claim 20, **characterized in that** the ratio between phospholipid/phospholipids and polyhydroxybutyrate in solution is from 8:2 up to 7:3.

**23.** The method of production according to claim 18, **characterized in that** the phospholipids are formed by lecithin and cholesterol in a ratio from 9:1 up to 1:1.
